# EUROPEAN PATENT APPLICATION

(11) **EP 2 997 878 A1**
(43) Date of publication of application: **23.03.2016**
(21) Application number: 13879701.4
(22) Date of filing: 26.09.2013
(51) Int. Cl.: A61B 1/04, A61B 1/00, A61B 1/06, A61B 90/00, A61M 25/09

(54) **ENDOSCOPIC SYSTEM**

(30) Priority: 27.03.2013 JP 2013066551
(71) Applicant: Olympus Corporation, Shibuya-ku Tokyo 151-0072 (JP)
(72) Inventor: YOSHIDA, Kazuhiro, Tokyo 151-0072 (JP)
(74) Representative: Schicker, Silvia
(86) International application number: PCT/JP2013/075982
(87) International publication number: WO 2014/155783

(57) **Abstract**

Provided is an endoscope system (100) including a light source device (3) that outputs signal light that penetrates tissue within a living body; a narrow medical instrument (1) that is inserted into a blood vessel and whose distal end has an emission window from which the signal light supplied from the light source device (3) is emitted; an endoscope (5) that is inserted into the living body and has an imager (13), which obtains an optical image by acquiring an image of the living body, and a detector (13), which detects the signal light emitted from the emission window; and an image processor (15) that generates a composite image by combining the optical image with a marker that indicates a position of the signal light.

## Description

### {Technical Field}

The present invention relates to endoscope systems.

### {Background Art}

In the related art, a known diagnostic device having a sound-wave generator at a distal end of a catheter externally detects a sound wave from the sound-wave generator and displays the sound-wave detected position on an X-ray image of an examination subject (for example, see Patent Literature 1). According to Patent Literature 1, the position of the distal end of the catheter inside the examination subject can be readily ascertained.

As a method for treating stenosis of a blood vessel in the heart, there is a known treatment method that involves piercing a guide wire through the stenosis site of the blood vessel while observing an X-ray image and then expanding the stenosis site with a balloon or a stent.

### {Citation List}

### {Patent Literature}

{PTL 1} Japanese Unexamined Patent Application, Publication No. Hei 05-161634

### {Summary of Invention}

### {Technical Problem}

However, the device in Patent Literature 1 is not suitable for treating a blood vessel in the heart since the position of the medical instrument inside the examination subject can only be roughly ascertained. In other words, when piercing the guide wire through the stenosis site, the guide wire needs to be pushed and pulled or rotated within the narrow blood vessel, thus requiring further accurate manipulation. In the device in Patent Literature 1, since the detailed positional relationship between the distal end of the guide wire and the blood vessel cannot be ascertained, it is difficult to accurately manipulate the guide wire within the blood vessel in the heart.

In view of the circumstances described above, an object of the present invention is to provide an endoscope system in which a medical instrument can be accurately manipulated while ascertaining the detailed position of the distal-end portion of the medical instrument within a blood vessel.

### {Solution to Problem}

In order to achieve the aforementioned object, the present invention provides the following solutions.

The present invention provides an endoscope system including a light source device that outputs signal light that penetrates tissue within a living body; a narrow medical instrument that is inserted into a blood vessel and whose distal end has an emission window from which the signal light supplied from the light source device is emitted; an endoscope that is inserted into the living body and has an imager, which obtains an optical image by acquiring an image of the living body, and a detector, which detects the signal light emitted from the emission window; and an image processor that generates a composite image by combining the optical image obtained by the imager with a marker that indicates a position of the signal light detected by the detector.

According to the present invention, when the signal light is supplied from the light source device to the medical instrument in a state where the endoscope is disposed at a position at which the distal-end portion of the medical instrument inserted into the blood vessel is included in the field of view of the optical image, the detector located outside the blood vessel detects the signal light emitted from the emission window, and the image processor generates the composite image having the marker added at the signal-light detected position. Consequently, based on the blood-vessel optical image and the marker clearly acquired with high magnification in the composite image, the operator can accurately manipulate the medical instrument while ascertaining the detailed position of the distal-end portion of the medical instrument within the blood vessel.

In the above invention, the light source device may output near-infrared light or infrared light as the signal light.

Accordingly, since near-infrared light or infrared light has high transmissivity through tissue, the quantity of signal light reaching the detector located outside the blood vessel from the emission window located inside the blood vessel increases, so that the signal light can be detected with high sensitivity.

In the above invention, the light source device may output visible light with a color different from a color of the living body as the signal light.

Accordingly, based on the difference in colors, the signal light and the living body can be readily distinguished from each other and be detected.

In the above invention, the imager may obtain the optical image when the signal light is emitted from the emission window and the optical image when the signal light is not emitted from the emission window. The image processor may generate the marker that indicates a region where a difference between the two optical images obtained by the imaging element is larger than or equal to a predetermined threshold value.

Accordingly, in a configuration in which the imager also functions as a detector, the imager can simultaneously acquire an image of the living body and an image of the signal light. Moreover, the signal light can be extracted with high precision from the optical image, and the marker can be added at a proper position.

In the above invention, the endoscope may include an illuminator that radiates illumination light onto the living body. The imager may obtain the two optical images in a state where the living body is not irradiated with the illumination light by the illuminator.

Accordingly, since the signal light can be detected with high sensitivity and a high S/N ratio by the imager, the signal light can be extracted with high precision from the optical image.

In the above invention, the medical instrument may include a light-guiding member that is disposed therein in a longitudinal direction and whose distal end has the emission window.

Accordingly, the signal light can be transmitted with a simple configuration from the base end to the distal end of the medical instrument.

In the above invention, the medical instrument may further have a plurality of the emission windows longitudinally arranged in a side surface of the medical instrument.

Accordingly, beams of signal light emitted from the plurality of emission windows are arrayed in conformity to the shape of the guide wire so that the shape of the guide wire within the blood vessel can be identified from an array of multiple markers in the composite image.

In the above invention, the medical instrument may further have a plurality of the emission windows circumferentially arranged in a side surface of the medical instrument.

Accordingly, the signal light can be constantly detected by the detector, regardless of the orientation of the medical instrument in the circumferential direction relative to the endoscope.

In the above invention, the medical instrument may have light-transmitting sections, which are capable of transmitting the signal light, at multiple positions in the longitudinal direction, and the light-guiding member may be provided in a movable manner in the longitudinal direction of the medical instrument.

Accordingly, by moving the distal end of the light-guiding member in the longitudinal direction of the medical instrument, the position of the signal light detected by the detector moves in the longitudinal direction of the medical instrument. Consequently, the shape of the guide wire within the blood vessel can be identified from the moving trajectory of the marker in the composite image.

In the above invention, the detector and the imager may include a common imaging element that acquires an image of light from the living body.

Accordingly, the positions of the optical image and the signal light can be readily associated with each other, thereby simplifying the composite-image generating process.

In the above invention, the endoscope system may further include an electrocardiographic signal detector that detects an electrocardiographic signal of the living body. The detector and the imager may respectively perform detection of the signal light and acquisition of the optical image with the same phase of a cycle of the electrocardiographic signal detected by the electrocardiographic signal detector.

Accordingly, when inserting the medical instrument into the blood vessel in the heart to observe the heart by using the endoscope, even if the heart is beating, the detection of the signal light and the acquisition of the optical image can be performed when the positional relationship of the heart is the same with respect to the detector and the imager.

In the above invention, the endoscope system may further include a notifying unit that provides a notification of an increase in intensity of the signal light detected by the detector.

Accordingly, the closer the distal end of the medical instrument is to a blood vessel wall, the higher the intensity of the signal light detected by the detector. Thus, for example, when the distal end of the medical instrument is in contact with the blood vessel wall, the operator can quickly recognize this condition based on a notification provided by the notifying unit.

In the above invention, the medical instrument may be a guide wire, a catheter, or an angioscope.

### {Advantageous Effects of Invention}

The present invention is advantageous in that a medical instrument can be accurately manipulated while ascertaining the detailed position of the distal-end portion of the medical instrument within a blood vessel.

### {Brief Description of Drawings}

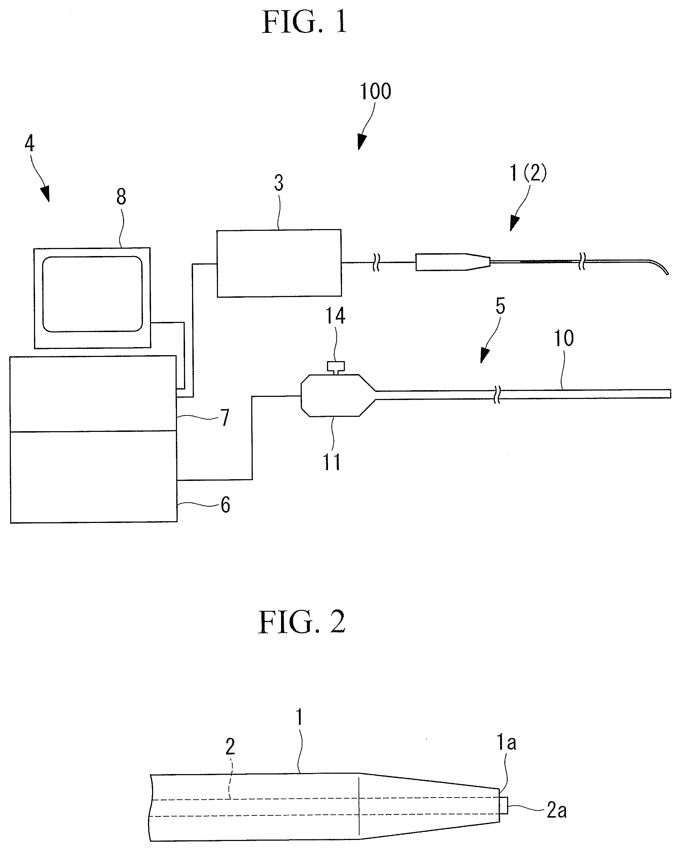

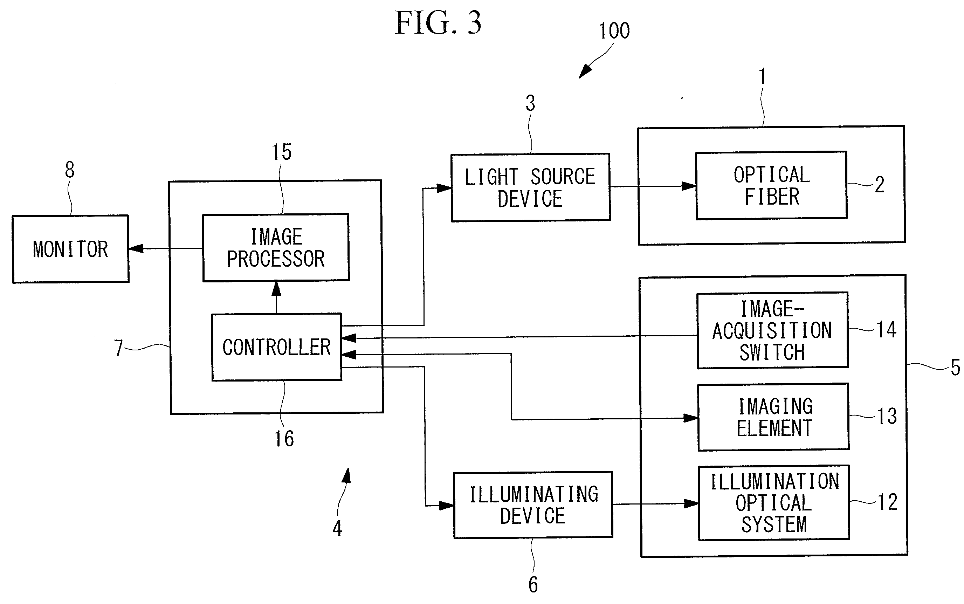

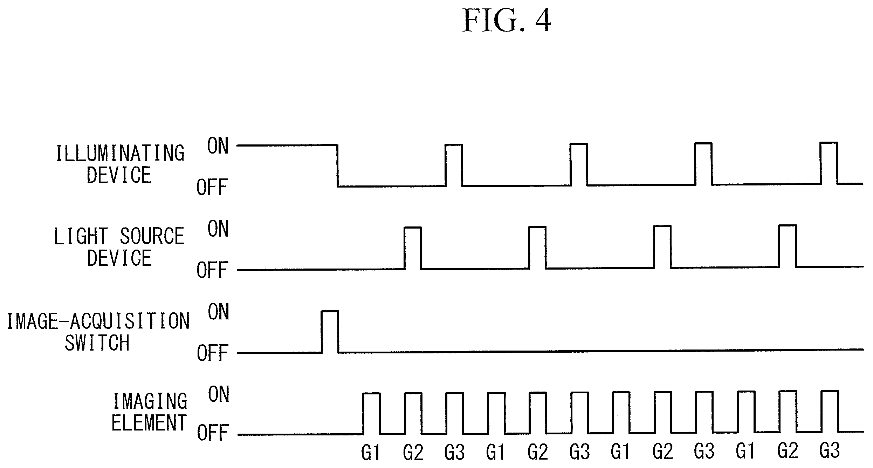

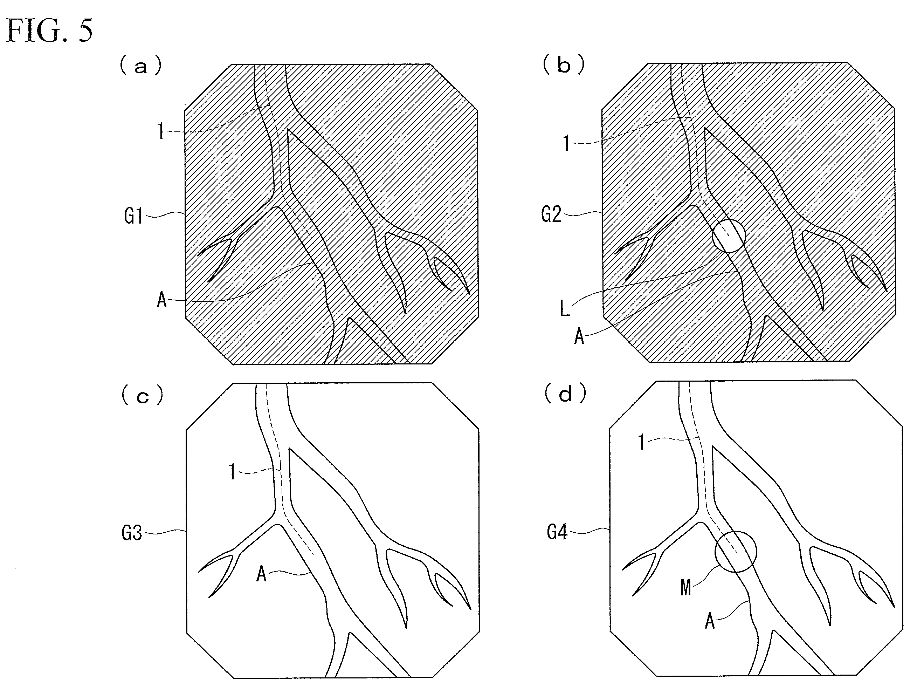

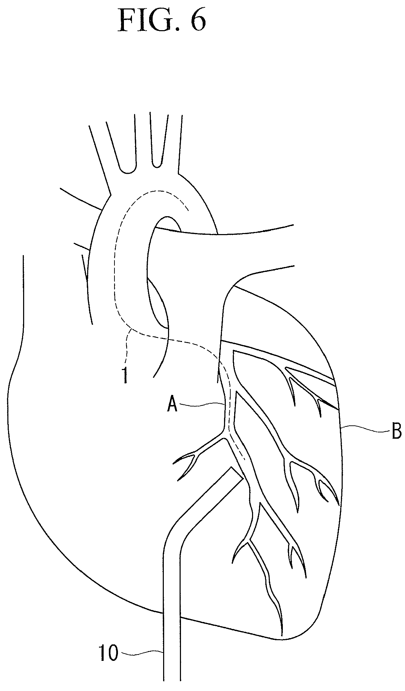

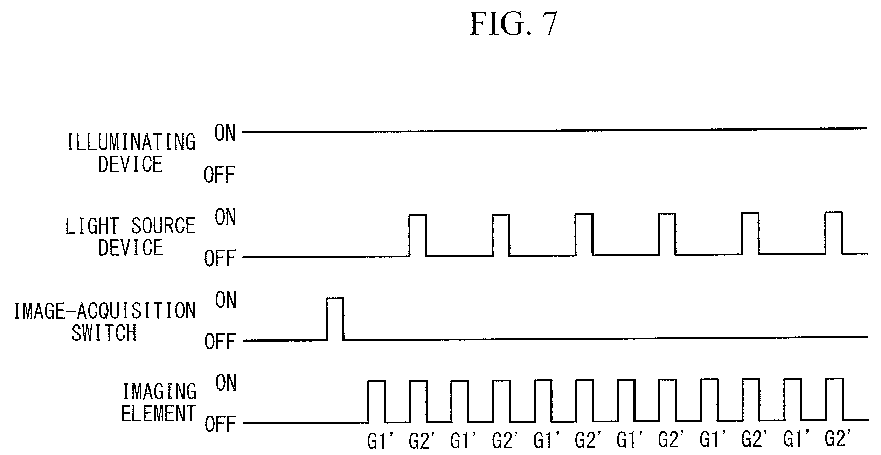

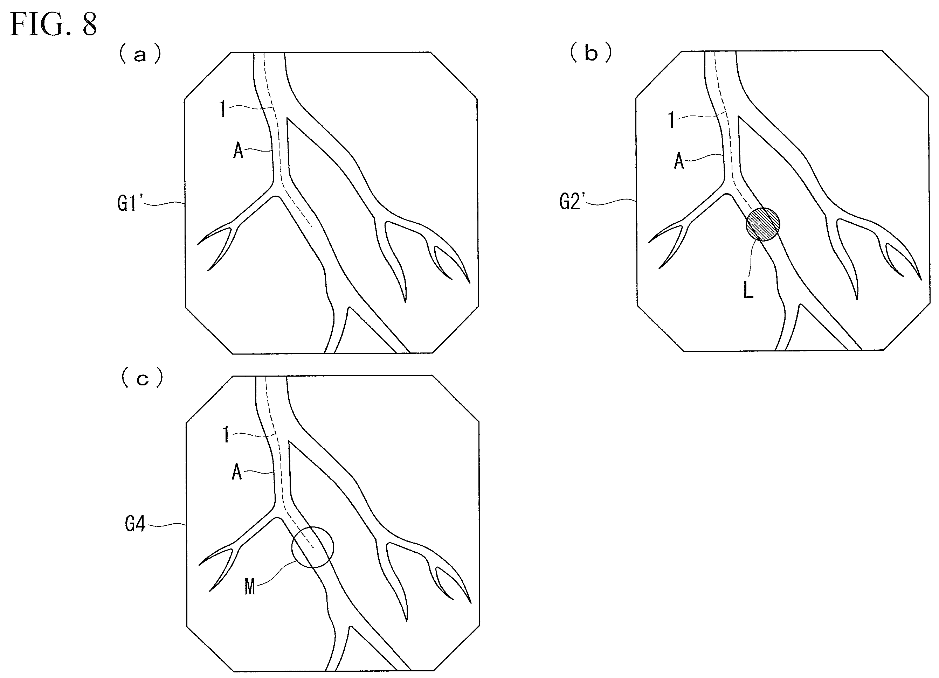

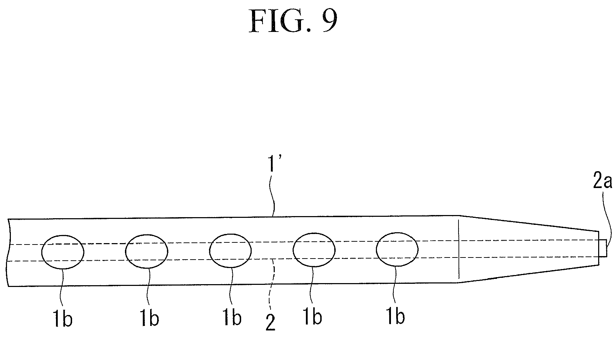

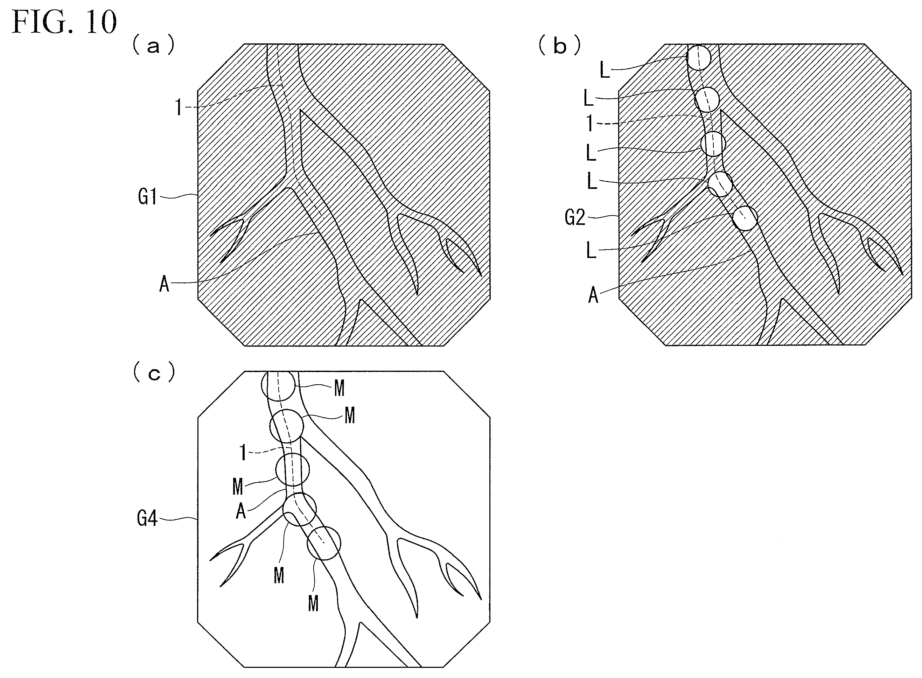

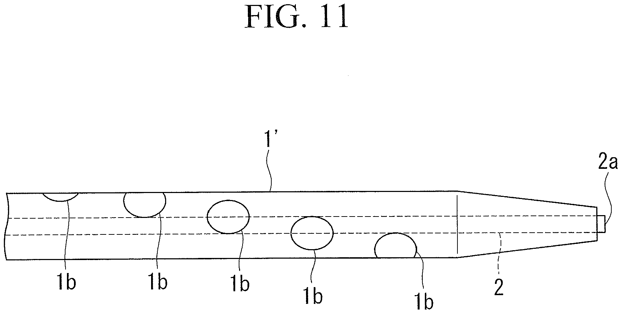

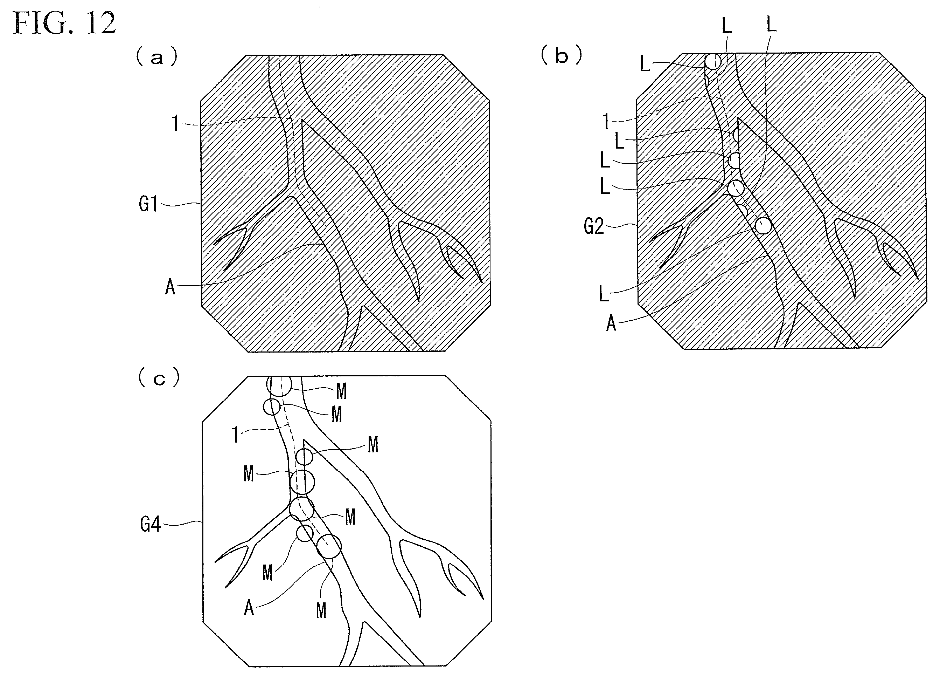

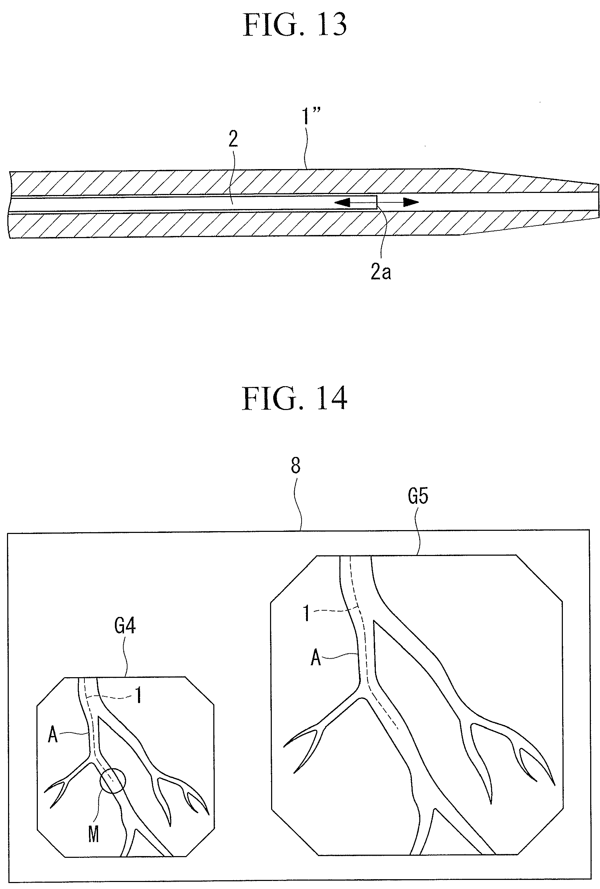

{Fig. 1} Fig. 1 is an external view illustrating the overall configuration of an endoscope system according to a first embodiment of the present invention.
{Fig. 2} Fig. 2 illustrates a distal-end portion of a guide wire included in the endoscope system in Fig. 1.
{Fig. 3} Fig. 3 is a functional block diagram illustrating the overall configuration of the endoscope system in Fig. 1.
{Fig. 4} Fig. 4 is a timing chart illustrating a composite-image acquisition operation of the endoscope system in Fig. 1.
{Fig. 5} Fig. 5 illustrates a first frame image (a), a second frame image (b), a third frame image (c), and a composite image (d) generated in the composite-image acquisition operation performed by the endoscope system in Fig. 1.
{Fig. 6} Fig. 6 illustrates a method of how an endoscope and the guide wire included in the endoscope system in Fig. 1 are used.
{Fig. 7} Fig. 7 is a timing chart illustrating a composite-image acquisition operation of an endoscope system according to a second embodiment of the present invention.
{Fig. 8} Fig. 8 illustrates a first frame image (a), a second frame image (b), and a composite image (c) generated in the composite-image acquisition operation in Fig. 7.
{Fig. 9} Fig. 9 illustrates a distal-end portion of a guide wire included in an endoscope system according to a third embodiment of the present invention.
{Fig. 10} Fig. 10 illustrates a first frame image (a), a second frame image (b), and a composite image (c) generated in the composite-image acquisition operation performed by using the guide wire in Fig. 9.
{Fig. 11} Fig. 11 illustrates a modification of the guide wire in Fig. 9.
{Fig. 12} Fig. 12 illustrates a first frame image (a), a second frame image (b), and a composite image (c) generated in the composite-image acquisition operation performed by using the guide wire in Fig. 11.
{Fig. 13} Fig. 13 illustrates a distal-end portion of a guide wire included in an endoscope system according to a fourth embodiment of the present invention.
{Fig. 14} Fig. 14 illustrates a modification of a display on a monitor.

### {Description of Embodiments}

### {First Embodiment}

An endoscope system 100 according to a first embodiment of the present invention will be described below with reference to Figs. 1 to 6.

As shown in Fig. 1, the endoscope system 100 according to this embodiment includes a guide wire (medical instrument) 1 having an optical fiber (light-guiding member) 2 therein, a light source device 3 that supplies light to the optical fiber 2, and an endoscopic device 4.

The guide wire 1 has a sufficiently small outside diameter and flexibility, allowing it to be inserted into a blood vessel in the heart. The optical fiber 2 is disposed within the guide wire 1, which is tubular, in the longitudinal direction thereof. As shown in Fig. 2, a distal end 2a of the optical fiber 2 slightly protrudes from a distal end 1a of the guide wire 1. Signal light emitted from the distal end (emission window) 2a of the optical fiber 2 spreads out in the vicinity of the distal-end portion of the guide wire 1, causing the distal-end portion of the guide wire 1 to glow. As an alternative to the distal end 2a of the optical fiber 2 protruding from the distal end 1a of the guide wire 1, a beam-spreading lens may be provided at the distal end 1a of the guide wire 1.

The light source device 3 outputs signal light with a wavelength that penetrates tissue, such as a blood vessel wall or fat, and supplies the signal light to a base end of the optical fiber 2.

The endoscopic device 4 includes an endoscope 5; an illuminating device 6 that supplies illumination light, which allows for white-light observation, to the endoscope 5; a video processor 7 that is connected to the light source device 3, the endoscope 5, and the illuminating device 6; and a monitor 8 that displays an image output from the video processor 7.

The endoscope 5 includes a narrow insertion section 10 and a manipulation section 11 provided at a base end of the insertion section 10.

The insertion section 10 has a sufficiently small outside diameter and flexibility, allowing it to be inserted into a space (pericardial cavity) between the heart and the pericardium that envelops the heart. As shown in Fig. 3, an illumination optical system (illuminator) 12 and an imaging element (detector, imager) 13 are provided at a distal end of the insertion section 10. The illumination optical system 12 radiates the illumination light supplied from the illuminating device 6 onto the living body. The imaging element 13 obtains an image of the illumination light reflected at the surface of the living body and an image of the signal light glowing at the distal-end portion of the guide wire 1, and transmits the obtained image information to the video processor 7.

The manipulation section 11 includes an image-acquisition switch 14 used for issuing a command to acquire a composite image G4, which will be described later, and outputs a composite-image generating signal to the video processor 7 when the image-acquisition switch 14 is pressed.

The video processor 7 includes an image processor 15 that generates an image from the image information acquired by the imaging element 13, and also includes a controller 16 that controls the light source device 3, the illuminating device 6, the imaging element 13, and the image processor 15.

In normal operation, the controller 16 causes the imaging element 13 to perform imaging in a state where illumination light is supplied from the illuminating device 6 to the endoscope 5, and sends image information received from the imaging element 13 to the image processor 15. The image processor 15 successively repeats a process of generating an endoscopic image (optical image), which is a white-light image of a living body, from the image information received from the controller 16 and outputting the endoscopic image to the monitor 8. Thus, the monitor 8 displays a real-time video of the endoscopic image of the living body.

When the controller 16 receives a composite-image generating signal from the endoscope 5, the controller 16 executes a composite-image acquisition operation for generating a composite image G4 in which a marker M indicating the position of the distal-end portion of the guide wire 1 is added to the endoscopic image.

This composite-image acquisition operation will be described with reference to Figs. 4 and 5.

When the image-acquisition switch 14 is pressed and the controller 16 receives a composite-image acquiring signal, the controller 16 first causes the imaging element 13 to perform imaging two consecutive times in a state where the supply of illumination light from the illuminating device 6 to the endoscope 5 is stopped, as shown in Fig. 4. In this case, the first imaging operation is executed in a state where signal light L is not supplied from the light source device 3 to the optical fiber 2, and the second imaging operation is executed in a state where the signal light L is supplied from the light source device 3 to the optical fiber 2. Thus, a first frame image G1 that is dark in its entirety, as shown in Fig. 5(a), and a second frame image G2 in which only a region irradiated with the signal light L corresponding to the distal-end portion of the guide wire 1 is bright, as shown in Fig. 5(b), are generated in the image processor 15. The images G1 to G4 in Figs. 5(a) to 5(d) are obtained as a result of acquiring images of a blood vessel A having the guide wire 1 inserted therein.

Then, the controller 16 causes the imaging element 13 to perform imaging in a state where illumination light is supplied from the illuminating device 6 to the endoscope 5. Thus, as shown in Fig. 5(c), a third frame image, which is a white-light image of the living body, is generated in the image processor 15.

The image processor 15 subtracts the first frame image G1 from the second frame image G2 and extracts a region where a difference in obtained gradation values is larger than or equal to a predetermined threshold value. Thus, the signal light L is extracted from the second frame image G2. The image processor 15 generates a marker M indicating the position of the extracted signal light L and combines the generated marker M with a third frame image G3, thereby generating a composite image G4, as shown in Fig. 5(d). Although a circle surrounding the extracted region of the signal light L is shown as the marker M in Fig. 5(d), the type of marker M is not limited to this and can be suitably changed.

Next, the operation of the endoscope system 100 having the above-described configuration will be described with reference to an example where a stenosis site of the blood vessel A in the heart B is treated.

When treating the stenosis site of the blood vessel A in the heart B by using the endoscope system 100 according to this embodiment, the operator first percutaneously inserts the insertion section 10 of the endoscope 5 into the pericardial cavity while observing the endoscopic image displayed on the monitor 8, and disposes the distal end of the insertion section 10 at a position where the stenosis site is observable, as shown in Fig. 6. Then, the operator inserts the guide wire 1 into the blood vessel A, which may be a coronary artery, in the heart B from, for example, the femoral artery or the femoral vein via the aorta, and introduces the guide wire 1 to the stenosis site while checking the position of the guide wire 1 on an X-ray image.

When the operator desires to check the position of the distal end of the guide wire 1 near the stenosis site in the blood vessel A, the operator presses the image-acquisition switch 14. Thus, the endoscope system 100 executes a composite-image acquisition operation by discontinuing normal operation and displays the composite image G4 on the monitor 8. In this case, the signal light L glowing at the distal-end portion of the guide wire 1 located within the blood vessel A penetrates the tissue and propagates to the outside of the heart B. The signal light L is then detected by the imaging element 13 and is indicated by a marker M in the composite image G4.

In the composite-image acquisition operation, both the emission and the detection of the signal light L are performed in the dark pericardial cavity where there is no external light or illumination light from outside the living body. Therefore, the signal light L glowing within the blood vessel A is detected with high sensitivity and a high S/N ratio by the imaging element 13 and is extracted with high accuracy from the second frame image G2.

The operator can recognize the position of the distal-end portion of the guide wire 1 within the blood vessel A from the position of the marker M in the composite image G4 displayed on the monitor 8. The operator pierces the guide wire 1 through the stenosis site of the blood vessel A, then inserts a stent or a balloon along the guide wire 1 to the stenosis site, and expands the stent or the balloon, so that the stenosis site can be treated.

Accordingly, in this embodiment, the position of the distal-end portion of the guide wire 1 located within the blood vessel A is indicated by the marker M in the composite image G4 in which an image of the blood vessel A is clearly acquired with high magnification. Therefore, the operator manipulates the guide wire 1 while carefully observing the position of the marker M added to the blood vessel A included in the composite image G4 so that, for example, the operator can clearly observe how the blood vessel wall bulges as a result of the distal end of the guide wire 1 strongly coming into contact with the blood vessel wall. This is advantageous in that the guide wire 1 can be properly manipulated while accurately ascertaining the positional relationship between the distal-end portion of the guide wire 1 and the blood vessel A.

In this embodiment, the video of the composite image G4 is displayed on the monitor 8 while successively generating the composite image G4. Alternatively, a still image may be displayed on the monitor 8 by generating the composite image G4 only once.

In this case, the endoscope system 100 may further include an electrocardiographic signal detector (not shown) that acquires an electrocardiographic signal. Based on the electrocardiographic signal, the controller 16 may determine the timings for acquiring the first, second, and third frame images G1, G2, and G3.

The image-acquisition range of the heart B by the imaging element 13 fluctuates in accordance with periodical beating of the heart B. The controller 16 causes the imaging element 13 to acquire the first, second, and third frame images G1, G2, and G3 with the same phase of the fluctuation cycle of the electrocardiographic signal. Thus, the field of view becomes the same for all of the first, second, and third frame images G1, G2, and G3. Accordingly, a composite image G4 with a marker M added at a more accurate position can be generated.

### {Second Embodiment}

Next, an endoscope system according to a second embodiment of the present invention will be described with reference to Figs. 7 and 8. In this embodiment, points different from those in the first embodiment will be mainly described. Components identical to those in the first embodiment will be given the same reference signs, and descriptions thereof will be omitted.

The endoscope system according to this embodiment differs from that in the first embodiment in that the light source device 3 supplies the optical fiber 2 with signal light L in a wavelength band different from that of the illumination light, and also in terms of the composite-image acquisition operation.

The light source device 3 supplies signal light L having a wavelength ranging between infrared wavelengths and near-infrared wavelengths to the optical fiber 2. Thus, the imaging element 13 used in the endoscope 5 has sensitivity to a wavelength band ranging between infrared wavelengths and near-infrared wavelengths. Since light with a wavelength ranging between infrared wavelengths and near-infrared wavelengths has high transmissivity through tissue, such light propagates with high efficiency from the inside of the blood vessel A to the outside of the heart B without being affected by, for example, a blood vessel wall or fat and is detected with high sensitivity by the imaging element 13.

As shown in Fig. 7, in the composite-image acquisition operation, the controller 16 causes the imaging element 13 to perform imaging two consecutive times in a state where the illuminating device 6 continuously supplies illumination light to the endoscope 5. In this case, the first imaging operation is executed in a state where the signal light L is not supplied from the light source device 3 to the optical fiber 2, and the second imaging operation is executed in a state where the signal light L is supplied from the light source device 3 to the optical fiber 2. Thus, a first frame image G1' and a second frame image G2' are generated in the image processor 15. As shown in Fig. 8(a), the first frame image G1' is a white-light image of a living body. As shown in Fig. 8(b), in the second frame image G2', a region irradiated with the signal light L corresponding to the distal-end portion of the guide wire 1 has a higher gradation value than the surrounding area.

The image processor 15 subtracts the first frame image G1' from the second frame image G2' and extracts a region where a difference in obtained gradation values is larger than or equal to a predetermined threshold value. Thus, the signal light L is extracted from the second frame image G2'. The image processor 15 generates a marker M indicating the position of the extracted signal light L and combines the generated marker M with the first frame image G1', thereby generating a composite image G4, as shown in Fig. 8(c).

In addition to the advantages of the first embodiment, the endoscope system according to this embodiment having the above-described configuration can readily detect signal light L distinctively from illumination light even when both the illumination light and the signal light L are radiated simultaneously onto the living body. Therefore, it is not necessary to repeat on-off operation of the illumination light, which is advantageous in that the control is simplified.

In this embodiment, signal light L having a wavelength ranging between infrared wavelengths and near-infrared wavelengths is used. Alternatively, visible light with a color different from that of the heart B may be used as the signal light L. For example, because the heart B contains large amounts of red and yellow colored tissue, green or blue light may be used as the signal light L. Accordingly, signal light L having a color different from that of the heart B in the second frame image G2' can be readily extracted from the second frame image G2'.

Furthermore, light with a wavelength to which the imaging element 13 has particularly high sensitivity may be used as the signal light L. Accordingly, since the signal light L has a higher gradation value in the second frame image G2', the signal light L can be readily extracted from the second frame image G2'.

### {Third Embodiment}

Next, an endoscope system according to a third embodiment of the present invention will be described with reference to Figs. 9 to 12. In this embodiment, points different from those in the first and second embodiments will be mainly described. Components identical to those in the first and second embodiments will be given the same reference signs, and descriptions thereof will be omitted.

As shown in Fig. 9, the endoscope system according to this embodiment differs from that in the first and second embodiments in that a guide wire 1' has a plurality of emission windows 1b longitudinally arranged in a side surface thereof.

The guide wire 1' emits signal light L simultaneously from the plurality of emission windows 1b in the side surface and from the distal end 2a of the optical fiber 2. When such a guide wire 1 is used, a second frame image G2 in which multiple beams of signal light L are arrayed along the blood vessel A is generated in the composite-image acquisition operation, as shown in Fig. 10(b).

The image processor 15 extracts the multiple beams of signal light L in the second frame image G2 by performing a process similar to that in the first and second embodiments, generates a plurality of markers M indicating the positions of the extracted beams of signal light L, and displays the markers M in a composite image G4, as shown in Fig. 10(c). In this case, the signal light L glowing at the distal-end portion of the guide wire 1 and the signal light L glowing in the emission windows 1b in the side surface may have different colors so as to be distinguishable from each other.

In addition to the advantages of the first embodiment, the endoscope system according to this embodiment having the above-described configuration is advantageous in that the traveling route of the guide wire 1 within the blood vessel A can be identified from the array of markers M arranged in a single line. Furthermore, since the operator can identify the shape of the guide wire 1 within the blood vessel A, the operator can check whether or not the side surface of the guide wire 1 is strongly in contact with the blood vessel wall by comparing the shape of the guide wire 1 with the shape of the blood vessel A.

In this embodiment, the multiple emission windows 1b provided in the side surface of the guide wire 1' may preferably be arranged spirally at different circumferential positions of the guide wire 1', as shown in Fig. 11.

Accordingly, an image of the signal light L emitted from any one of the emission windows 1b can be constantly acquired by the imaging element 13, regardless of the orientation of the guide wire 1' within the blood vessel in the circumferential direction.

Furthermore, in a case where the emission windows 1b are spirally arranged, the signal light L and the markers M move in the rotational direction of the guide wire 1' when the guide wire 1' is rotated in the circumferential direction within the blood vessel A, as shown in Figs. 12(a) to 12(c), so that the orientation of the guide wire 1' in the circumferential direction can be identified from this movement. Therefore, when the guide wire 1' used has a bent distal-end portion, the distal end of the guide wire 1' can be readily led in a desired direction at a position where the blood vessel A branches off into multiple segments, so that the guide wire 1' can be manipulated more accurately.

### {Fourth Embodiment}

Next, an endoscope system according to a fourth embodiment of the present invention will be described with reference to Fig. 13. In this embodiment, points different from those in the first to third embodiments will be mainly described. Components identical to those in the first to third embodiments will be given the same reference signs, and descriptions thereof will be omitted.

As shown in Fig. 13, the endoscope system according to this embodiment differs from that in the first to third embodiments in that the optical fiber 2 is provided within a guide wire 1" in a longitudinally movable manner.

The guide wire 1" has light-transmitting sections, which can transmit signal light L, at multiple positions in the longitudinal direction. The light-transmitting sections are constituted of the main body of the guide wire 1" composed of a transparent material relative to the signal light L. Alternatively, the guide wire 1" may be formed by winding a narrow wire into a spiral, and the light-transmitting sections may be constituted of gaps between wire sections adjacent to each other in the longitudinal direction.

With the endoscope system according to this embodiment having the above-described configuration, when the operator moves the optical fiber 2 in the longitudinal direction within the guide wire 1", the position of the marker M in the composite image G4 moves in conformity to the shape of the guide wire 1". Therefore, in addition to the advantages of the first embodiment, this embodiment is advantageous in that the shape of the blood vessel A can be ascertained more accurately since the distal end 2a of the optical fiber 2 at which the intensity of the signal light L is high can be moved.

In the first to fourth embodiments, the composite image G4 is displayed on the monitor 8 in place of an unprocessed endoscopic image during the composite-image acquisition operation. Alternatively, as shown in Fig. 14, the composite image G4 may be displayed next to an unprocessed endoscopic image G5.

Accordingly, by viewing the unprocessed endoscopic image G5, the operator can observe the blood vessel A without being disturbed by the marker M.

In this case, the composite image G4 may be displayed as a still image or as a video repeatedly played back over a short period.

Because the relative position between the heart B and the distal end of the insertion section 10 fluctuates in accordance with the beating of the heart B, the field of view of the endoscopic image also fluctuates, sometimes causing the region of interest to shift from the field of view of the endoscopic image. In this case, the operator can constantly check the region of interest by referring to the composite image G4.

Furthermore, in the first to fourth embodiments, a notifying unit that provides a notification of an increase in intensity of the signal light L extracted in the image processor 15 may be further provided. For example, the image processor 15 may change the mode of the marker M to be generated in accordance with the gradation value of the signal light L.

The closer the distal end of the guide wire 1, 1', or 1" is to a blood vessel wall, the higher the intensity of the signal light L detected by the imaging element 13. Furthermore, when a blood vessel wall is locally thin due to being pressed by the distal end of the guide wire 1, the intensity of the signal light L detected by the imaging element 13 further increases. The operator can quickly recognize such conditions from a change in the mode of the marker M in the composite image G4 and can manipulate the guide wire 1, 1', or 1" more appropriately.

Furthermore, although the guide wires 1, 1', and 1" are described as examples of medical instruments in the first to fourth embodiments, the configuration described in each embodiment is applicable to other medical instruments used by being inserted into blood vessels, such as a catheter or an angioscope.

### {Reference Signs List}

- 100: endoscope system
- 1: guide wire
- 1b: emission window
- 2: optical fiber
- 2a: distal end (emission window)
- 3: light source device
- 4: endoscopic device
- 5: endoscope
- 6: illuminating device
- 7: video processor
- 8: monitor
- 10: insertion section
- 11: manipulation section
- 12: illumination optical system (illuminator)
- 13: imaging element (imager, detector)
- 14: image-acquisition switch
- 15: image processor
- 16: controller
- A: blood vessel
- B: heart
- L: signal light
- M: marker

## Claims

1. An endoscope system comprising:
a light source device that outputs signal light that penetrates tissue within a living body;
a narrow medical instrument that is inserted into a blood vessel and whose distal end has an emission window from which the signal light supplied from the light source device is emitted;
an endoscope that is inserted into the living body and has an imager, which obtains an optical image by acquiring an image of the living body, and a detector, which detects the signal light emitted from the emission window; and
an image processor that generates a composite image by combining the optical image obtained by the imager with a marker that indicates a position of the signal light detected by the detector.

2. The endoscope system according to Claim 1,
wherein the light source device outputs near-infrared light or infrared light as the signal light.

3. The endoscope system according to Claim 1,
wherein the light source device outputs visible light with a color different from a color of the living body as the signal light.

4. The endoscope system according to any one of Claims 1 to 3,
wherein the imager obtains the optical image when the signal light is emitted from the emission window and the optical image when the signal light is not emitted from the emission window, and
wherein the image processor generates the marker that indicates a region where a difference between the two optical images obtained by the imager is larger than or equal to a predetermined threshold value.

5. The endoscope system according to Claim 4,
wherein the endoscope includes an illuminator that radiates illumination light onto the living body, and
wherein the imager obtains the two optical images in a state where the living body is not irradiated with the illumination light by the illuminator.

6. The endoscope system according to any one of Claims 1 to 5,
wherein the medical instrument includes a light-guiding member that is disposed therein in a longitudinal direction and whose distal end has the emission window.

7. The endoscope system according to any one of Claims 1 to 6,
wherein the medical instrument further has a plurality of the emission windows longitudinally arranged in a side surface of the medical instrument.

8. The endoscope system according to any one of Claims 1 to 7,
wherein the medical instrument further has a plurality of the emission windows circumferentially arranged in a side surface of the medical instrument.

9. The endoscope system according to Claim 6,
wherein the medical instrument has light-transmitting sections, which are capable of transmitting the signal light, at multiple positions in the longitudinal direction, and
wherein the light-guiding member is provided in a movable manner in the longitudinal direction of the medical instrument.

10. The endoscope system according to any one of Claims 1 to 9,
wherein the detector and the imager include a common imaging element that acquires an image of light from the living body.

11. The endoscope system according to any one of Claims 1 to 10, further comprising:
an electrocardiographic signal detector that detects an electrocardiographic signal of the living body,
wherein the detector and the imager respectively perform detection of the signal light and acquisition of the optical image with the same phase of a cycle of the electrocardiographic signal detected by the electrocardiographic signal detector.

12. The endoscope system according to any one of Claims 1 to 11, further comprising:
a notifying unit that provides a notification of an increase in intensity of the signal light detected by the detector.

13. The endoscope system according to any one of Claims 1 to 12,
wherein the medical instrument is a guide wire, a catheter, or an angioscope.
